# EUROPÄISCHE PATENTANMELDUNG

(11) **EP 1 282 062 A2**
(43) Veröffentlichungstag der Anmeldung: **05.02.2003**
(21) Anmeldenummer: 02016431.5
(22) Anmeldetag: 22.07.2002
(51) Int. Cl.: G06F 19/00

(54) **Verfahren für die Vermittlung einer telemedizinischen Gesundheitsdienstleistung**

(30) Priorität: 31.07.2001 DE 10137430
(71) Anmelder: SIEMENS AKTIENGESELLSCHAFT, 80333 München (DE)
(72) Erfinder: Christ, Tilo, 91058 Erlangen (DE); Prihoda, Heinz, 90562 Heroldsberg (DE); Schmidt, Volker, Dr., 91054 Erlangen (DE); Schneider, Siegfried, Dr., 91056 Erlangen (DE); Schüll, Hans-Dieter, 91085 Weisendorf (DE); Striebel, Werner, 90592 Schwarzenbruck (DE); Zahlmann, Gudrun, Dr., 92318 Neumarkt (DE)

(57) **Zusammenfassung**

Die Erfindung betrifft ein Verfahren zur automatischen Überprüfung, ob zwischen einem Leistungsanbieter einer telemedizinischen Gesundheitsdienstleistung und einem Kunden ein Vertrag über die telemedizinische Gesundheitsdienstleistung zustande kommen kann. Im Zuge des Verfahrens wird von einer Datenverarbeitungseinrichtung (4) eines Vermittlers eine Anfrage eines Kunden zu einer telemedizinischen Gesundheitsdienstleistung angenommen. Daraufhin kontaktiert die Datenverarbeitungseinrichtung (4) eine erste Datenbank (5), welche Informationen über Leistungsangebote von Leistungsanbietern telemedizinischer Gesundheitsdienstleistungen enthält, und überprüft, ob der Anfrage ein im Wesentlichen entsprechendes Leistungsangebot eines Leistungsanbieters zugeordnet werden kann.

## Beschreibung

Die Erfindung betrifft ein Verfahren für die Vermittlung einer telemedizinischen Gesundheitsdienstleistung zwischen einem Leistungsanbieter einer telemedizinischen Gesundheitsdienstleistung und einem Kunden.

Bei der Vermittlung telemedizinischer Gesundheitsdienstleistungen, worunter jegliche Arten von Telemedizin, also beispielsweise die Befundung von medizinischen Datensätzen, welche an eine Dienstleistungseinrichtung für telemedizinische Gesundheitsdienstleistungen über ein Kommunikationsnetz übersandt wurden, oder die Online-Befundung von Körperfunktionen unter Zuhilfenahme von technischen Mitteln zur Fernübertragung von Daten, verstanden wird, kommen in der Regel zwischen Leistungsanbietern und Kunden solcher Gesundheitsdienstleistungen Verträge zustande. Häufig ist es jedoch schwierig für die Bedürfnisse des Kunden einen geeigneten Leistungsanbieter als Vertragspartner zu finden.

Weitere Probleme können dann auftreten, wenn der Kunde und der Leistungsanbieter einer telemedizinischen Gesundheitsdienstleistung in unterschiedlichen Ländern, also in unterschiedlichen Rechtsräumen, lokalisiert sind. Dann ist häufig unklar, ob die telemedizinische Gesundheitsdienstleistung in dem Land des Kunden aufgrund gesetzlicher Vorschriften überhaupt erbracht werden darf und inwieweit derartige telemedizinische Gesundheitsdienstleistungen von der Krankenkasse des Kunden übernommen werden. Beispielsweise könnte sich eine Situation ergeben, in der ein in England arbeitender Arzt telemedizinische Gesundheitsdienstleistungen in Holland für einen dort zu Besuch befindlichen Patienten mit griechischer Nationalität anbietet, bei der die angedeuteten Fragen der rechtlichen Zulässigkeit sowie der Abrechenbarkeit der Gesundheitsdienstleistung möglichst schnell zu klären sind. Dies stößt in der Praxis immer wieder auf große Probleme, da die für die Erbringung telemedizinischer Gesundheitsdienstleistungen erforderlichen rechtlichen Bedingungen und Qualitätsrahmenbedingungen ggf. landesspezifisch unterschiedlich sind. Exemplarisch hierfür sei das Mammographiescreening genannt, welches in Holland und Deutschland unterschiedlich gehandhabt wird.

In der US 5,835,897 ist ein Verfahren beschrieben, welches Managern im Gesundheitswesen helfen soll, die Qualität und das Preis-/Leistungsverhältnis von Dienstleistungsanbietern im Gesundheitswesen bestimmen und vergleichen zu können. Dabei werden Patienten nach ihrer Krankheit und deren Behandlung klassifiziert. Basierend auf dieser Klassifizierung können die Leistungen verschiedener Dienstleistungsanbieter zur Behandlung der Krankheit verglichen werden.

Der Erfindung liegt die Aufgabe zugrunde, ein Verfahren für die Vermittlung einer telemedizinischen Gesundheitsdienstleistung derart anzugeben, dass die Überprüfung, ob zwischen einem Leistungsanbieter einer telemedizinischen Gesundheitsdienstleistung und einem Kunden überhaupt ein Vertrag über die Gesundheitsdienstleistung geschlossen werden kann, vereinfacht und in verhältnismäßig kurzer Zeit durchgeführt wird.

Nach der Erfindung wird diese Aufgabe gelöst durch ein Verfahren zu einer automatischen Überprüfung durch eine Datenverarbeitungseinrichtung, ob zwischen einem Leistungsanbieter einer telemedizinischen Gesundheitsdienstleistung und einem Kunden ein Vertrag über die telemedizinische Gesundheitsdienstleistung zustande kommen kann, aufweisend folgende Verfahrensschritte:
a) Annahme einer Anfrage eines Kunden zu einer telemedizinischen Gesundheitsdienstleistung durch eine an einem öffentlichen Kommunikationsnetz angeschlossene Datenverarbeitungseinrichtung, welche Anfrage von einem Rechner des Kunden über das öffentliche Kommunikationsnetz an die Datenverarbeitungseinrichtung übermittelt wird,
b) Kontaktierung einer ersten Datenbank durch die Datenverarbeitungseinrichtung, welche Informationen über Leistungsangebote von Leistungsanbietern telemedizinischer Gesundheitsdienstleistungen enthält, und
c) Überprüfung durch die Datenverarbeitungseinrichtung, ob der Anfrage zu der telemedizinischen Gesundheitsdienstleistung ein im Wesentlichen entsprechendes Leistungsangebot eines Leistungsanbieters zugeordnet werden kann.

Das erfindungsgemäße Verfahren erlaubt es einem Vermittler telemedizinischer Gesundheitsdienstleistungen automatisiert durch eine Datenverarbeitungseinrichtung auf in einer ersten Datenbank gespeicherte Informationen über Leistungsangebote von verschiedenen Leistungsanbietern telemedizinischer Gesundheitsdienstleistungen zurückzugreifen und automatisiert zu überprüfen, ob ein Leistungsangebot existiert, welches sich der Anfrage eines Kunden zuordnen lässt. Der Vermittler bzw. die Vermittlungsstelle kann also in verhältnismäßig kurzer Zeit überprüfen, ob ein geeignetes Leistungsangebot vorliegt und demnach ob zwischen einem Leistungsanbieter einer telemedizinischen Gesundheitsdienstleistung und dem Kunden ein Vertrag über die Gesundheitsdienstleistung geschlossen werden kann. Bei einer adäquaten Übereinstimmung zwischen der Anfrage des Kunden und einem Leistungsanbot eines Leistungsanbieters können der Kunde, der Leistungsanbieter oder beide Seiten informiert werden, damit Verhandlungen über den Abschluss eines Vertrages zur Erbringung der telemedizinischen Gesundheitsdienstleitung aufgenommen werden können. Medizinische Einrichtungen, wie Ärztepraxen und Kliniken, oder einzelne Ärzte können sowohl Leistungsanbieter für telemedizinische Gesundheitsdienstleitungen als auch Kunden für telemedizinische Gesundheitsdienstleistungen sein. Als Kunde kommt auch der einzelne Patient in Frage. Die Datenverarbeitungseinrichtung, welche die Funktion eines vollautomatischen Vertragsprüfungsmoduls hat, ist dabei an ein öffentliches Kommunikationsnetz, beispielsweise das Internet, angeschlossen, so dass das Vertragsprüfungsmodul praktisch für jedermann erreichbar ist.

Eine Ausführungsform der Erfindung sieht vor, dass die Datenverarbeitungseinrichtung im Zuge des Verfahrens eine zweite Datenbank kontaktiert, welche länderspezifische rechtliche Informationen zu telemedizinischen Gesundheitsdienstleistungen enthält. Auf diese Weise kann geklärt werden, welche telemedizinischen Leistungen in welchen Ländern erlaubt sind, und welche standesrechtrechtlichen Probleme in welchen Ländern existieren. Demnach kann unmittelbar und schnell festgestellt werden, ob die Erbringung einer telemedizinischen Gesundheitsdienstleistung für einen Kunden in einem bestimmten Land rechtlich zulässig ist.

Eine andere Ausführungsform der Erfindung sieht vor, dass die Datenverarbeitungseinrichtung im Zuge des Verfahrens eine dritte Datenbank kontaktiert, welche Informationen darüber enthält, welche telemedizinischen Gesundheitsdienstleistungen von Krankenkassen unter welchen Bedingungen zumindest teilweise bezahlt oder welche nicht bezahlt werden. Der Kunde erfährt also durch diese Abfrage insbesondere welche Bezahlungen bzw. Zuzahlungen durch seine Krankenkasse möglich sind, ob ein Vergütungssplitting, also eine Aufteilung der mit der Krankenkasse abrechenbaren, medizinischen Leistung auf zwei oder mehrere Leistungserbringer, zulässig ist und welche qualitativen Grundbedingungen hierfür erfüllt sein müssen.

Eine besonders bevorzugte Ausführungsform der Erfindung sieht vor, dass in dem Fall, dass der Anfrage eines Kunden ein Leistungsangebot eines Leistungsanbieters zugeordnet werden kann, zumindest ein Rohvertrag über die telemedizinische Gesundheitsdienstleistung durch die Datenverarbeitungseinrichtung erzeugt wird. Der Rohvertrag im Sinne eines Vertragsentwurfes dient in der Regel als Basis für den endgültig zwischen den Parteien zu schließenden Vertrag. Gegebenenfalls kann der Rohvertrag, soweit alle Vertragsbedingungen bereits bekannt und in dem Rohvertrag enthalten sind, auch der endgültige Vertrag sein.

Varianten der Erfindung sehen vor, dass die Anfrage eines Kunden eine medizinische Fragestellung und Identifikationsdaten des Kunden enthält, und dass die in der ersten Datenbank enthaltenen Leistungsangebote die Art und den Umfang der medizinischen Leistung sowie Identifikationsdaten des Anbieter enthalten. Hinsichtlich der medizinischen Fragestellung des Kunden, bei der es sich um einen Erst- oder Zweitbefund oder bei der es sich um die Überwachung von Körperfunktionen handeln kann, insbesondere hinsichtlich deren Formulierung, kann der Kunde durch geeignete Hilfsmittel, beispielsweise durch vom Kunden zu beantwortende Hilfsfragen, unterstützt werden, um zu einer eindeutigen, verständlichen und suchbaren medizinischen Fragestellung zu gelangen. Die Identifikationsdaten des Kunden beinhalten vorzugsweise alle für einen Vertragsschluss erforderlichen persönlichen Daten, beispielsweise den Namen, das Geburtsdatum, sofern vorhanden die Krankenkasse und die Krankenversicherungsnummer des Kunden. Die Identifikationsdaten des Leistungsanbieters enthalten in entsprechender Weise alle für einen Vertragsschluss erforderlichen Daten des Leistungsanbieters, also beispielsweise Name und Anschrift des Leistungsanbieters sowie Kassenzulassung. Auf diese Weise kann ohne längere Kommunikation zwischen dem Kunden, dem Vermittler und dem Leistungsanbieter ein Rohvertrag erstellt werden.

Ausführungsformen der Erfindung sehen vor, dass durch die Datenverarbeitungseinrichtung durch Abfragen der ersten Datenbank überprüft wird, ob ein Leistungsanbieter in der Anfrage eines Kunden enthaltene qualitative und/oder quantitative Vorgaben für die zu erbringende telemedizinische Gesundheitsdienstleitung einhalten kann. Dies ist vor allem dann von Bedeutung, wenn eine größere medizinische Einrichtung, beispielsweise eine Spezialklinik, für einen bestimmten Zeitraum die Erbringung einer telemedizinischen Gesundheitsdienstleitung mit einer vorgegebenen Qualität wünscht.

Eine weitere Ausführungsform der Erfindung sieht vor, dass die Datenverarbeitungseinrichtung nach Abschluss eines Vertrages basierend auf dem Vertragsinhalt Arbeitsanweisungen und/oder Leitlinien für den Kunden und den Leistungsanbieter erzeugt, um einen reibungslosen Ablauf bei der Erbringung der telemedizinischen Gesundheitsdienstleistung zu ermöglichen. Die Erzeugung Arbeitsanweisungen und Leitlinien ist besonders einfach, wenn der Vertrag dem Rohvertrag entspricht, so dass der Datenverarbeitungseinrichtung nur mitgeteilt werden muss, dass der Vertrag zustande gekommen ist. Andernfalls müssen der Datenverarbeitungseinrichtung die geänderten Vertragsinhalte übermittelt werden, so dass die Datenverarbeitungseinrichtung die geänderten Vertragsbedingungen bei der Erzeugung der Arbeitsanweisungen und Leitlinien berücksichtigen kann.

Gemäß einer Variante der Erfindung ist die Datenverarbeitungseinrichtung als Expertensystem ausgebildet, welche wissensbasiert unter Berücksichtigung aller rechtlicher, abrechnungstechnischer und qualitativer Randbedingungen überprüft, ob ein Vertragsschluss möglich ist und gegebenenfalls zumindest einen Rohvertrag erstellt.

Gemäß Varianten der Erfindung sind auch die drei Datenbanken an ein öffentliches Kommunikationsnetz angeschlossen. Bei den Datenbanken muss es sich demnach nicht notwendigerweise um lokale Datenbanken handeln.

Ein Ausführungsbeispiel der Erfindung ist in der beigefügten schematischen Zeichnung dargestellt, welche eine Kommunikationsstruktur zur Ausführung des erfindungsgemäßen Verfahrens zeigt.

Die Kommunikationsstruktur weist im Falle des vorliegenden Ausführungsbeispiels ein öffentliches Kommunikationsnetz 1 auf, an das ein Rechner 2, beispielsweise ein PC, eines Kunden, eine Rechner 3, beispielsweise ein PC, eines Leistungsanbieters, eine Datenverarbeitungseinrichtung 4 eines Vermittlers von telemedizinischen Gesundheitsdienstleistungen und drei Datenbanken 5 bis 7 angeschlossen sind. Bei dem Kommunikationsnetz 1 handelt es sich im Falle des vorliegenden Ausführungsbeispiels um das Internet. Die Datenverarbeitungseinrichtung 4 ist ein Vertragsprüfungsmodul, welches als Expertensystem ausgebildet ist, also wissensbasiert arbeitet.

Die Datenbank 5 ist eine sogenannte Leistungsdatenbank, welche Informationen über telemedizinische Leistungsangebote von verschiedenen Leistungsanbietern enthält. Insbesondere enthält die Datenbank Informationen darüber, welche Art von telemedizinischen Gesundheitsdienstleistungen, in welcher Qualität diese, also beispielsweise ob Fachärzte zur Verfügung stehen oder in welcher Weise die Befundung erfolgt, in welcher Anzahl pro Zeiteinheit und in welchem Umfang telemedizinische Gesundheitsdienstleistungen von den Leistungsanbietern erbracht werden, also welche telemedizinischen Leistungen in einem Leistungspaket eines Leistungsanbieters enthalten sind. Darüber hinaus enthält die Datenbank 5 Informationen über erforderliche Eingabedaten für die Prüfung einer Anfrage eines Kunden, beispielsweise über die Anamnese, also die Vorgeschichte einer Krankheit, über existierende Befunde oder über vorhandene diagnostische Bildinformationen und deren Qualität.

Die Datenbank 6 ist eine Rechtsdatenbank, welche länderspezifische rechtliche Informationen zu telemedizinischen Gesundheitsdienstleistungen enthält. Hier sind insbesondere Informationen darüber gespeichert, in welchen Ländern welche telemedizinischen Gesundheitsdienstleistungen erlaubt sind und demnach erbracht werden dürfen. Darüber hinaus enthält die Datenbank 6 landesspezifische standesrechtliche Informationen in bezug auf die Erbringung medizinischer, insbesondere telemedizinischer Gesundheitsdienstleistungen.

Die Datenbank 7 ist eine sogenannte Abrechnungsdatenbank, welche Informationen darüber enthält, welche telemedizinischen Gesundheitsdienstleistungen von welcher Krankenkasse in welchem Land und unter welchen Bedingungen zumindest anteilig bezahlt oder nicht bezahlt werden.

Im Falle des vorliegenden Ausführungsbeispiels leidet der Kunde oder auch Patient, welcher deutscher Staatsbürger ist und seinen Wohnsitz, welcher auch Leistungsort sein soll, in Deutschland hat, an einer bereits diagnostizierten Krankheit, welche eine regelmäßige Überprüfung der Blutgerinnungswerte des Kunden mit einer entsprechenden medizinischen Überwachung des Krankheitsverlaufes erfordert, was telemedizinisch erfolgen kann. Der Kunde, welcher einen geeigneten Leistungsanbieter einer Gesundheitsdienstleistung sucht, der diese Leistungen erbringt, ruft unter Verwendung seines Rechners 2, eines geeigneten Netzwerkbrowsers, beispielsweise des Programms Netscape® Communicator 4.5 der Firma Netscape Communications Corporation, und der entsprechenden Internetadresse über das Internet 1 die sogenannte Homepage des Vertragsprüfungsmoduls 4 des Vermittlers von telemedizinischen Gesundheitsdienstleistungen auf, worunter eine Startseite eines Computerprogramms verstanden wird, welches eine Anfrage des Kunden hinsichtlich der von ihm erwünschten telemedizinischen Gesundheitsdienstleistung behandelt.

Mit Hilfe des Computerprogramms werden vorzugsweise unter Anleitung des Computerprogramms einerseits Identifikationsdaten des Kunden aufgenommen und andererseits sofern nicht schon vom Kunden entsprechend formuliert, eine medizinische Fragestellung in bezug auf die Überwachung der Blutgerinnungswerte formuliert, welche derart spezifiziert ist, dass in der Datenbank 5 nach einem Leistungsangebot eines Leistungsanbieters gesucht werden kann. Dabei werden auch Informationen zu existierenden Befunden, zum Krankheitsverlauf sowie zu Leistungswünsche des Kunden, beispielsweise vollständige Bezahlung der Gesundheitsdienstleistung durch seine Krankenkassen und die Bereitstellung eines Gerätes zur automatisierten Messung der Blutgerinnungswerte, aufgenommen.

Liegen die Identifikationsdaten des Kunden vor, bei denen es sich in der Regel zumindest um den Namen, die Anschrift, das Geburtsdatum sowie sofern vorhanden um die Krankenkasse und die Krankenversicherungsnummer des Kunden handelt, und ist die medizinische Fragestellung hinreichend spezifiziert sucht das Vertragsprüfungsmodul 4 in der Datenbank automatisch nach einer passenden telemedizinischen Gesundheitsdienstleistung in dem Leistungsangebot verschiedener Leistungsanbieter. Gesucht wird dabei nach Leistungsangeboten, welche zumindest im Wesentlichen die Wünsche des Kunden hinsichtlich der telemedizinischen Gesundheitsdienstleistung zur Überwachung der Blutgerinnungswerte erfüllen. Die Leistungsanbieter der telemedizinischen Gesundheitsdienstleistungen haben ihren Sitz in bzw. bieten ihre Gesundheitsdienstleistungen in der Regel aus verschiedenen Ländern an.

Im Falle des vorliegenden Ausführungsbeispiels hat das Computerprogramm des Vertragsprüfungsmoduls 4 drei geeignete, den Wünschen des Kunden entsprechende Leistungsangebote von drei verschiedenen Leistungsanbietern gefunden. Einer der Leistungsanbieter hat seinen Firmensitz in Deutschland, einer in USA und einer in Australien. Anschließend prüft das Computerprogramm des Vertragsprüfungsmoduls 4 automatisch anhand der in der Datenbank 6 vorhanden rechtlichen Informationen, ob aufgrund der Nationalität des Kunden, des Wohnsitzes und des Leistungsortes des Kunden sowie aufgrund des Sitzes der in Frage kommenden Leistungsanbieter rechtliche Probleme hinsichtlich der Erbringung der gewünschten telemedizinischen Gesundheitsdienstleistung auftreten können. Vorliegend existieren keinerlei derartige rechtlichen Probleme. Demnach könnte jeder der ermittelten Leistungsanbieter mit Sitz in Deutschland, USA bzw. Australien aus rechtlicher Sicht die telemedizinische Gesundheitsdienstleistung für den Kunden erbringen.

Des weiteren prüft das Computerprogramm des Vertragsprüfungsmoduls 4 die Zahlungssituation der telemedizinischen Gesundheitsdienstleistung zur Überwachung der Blutgerinnungswerte für den Kunden anhand der in der Datenbank 7 enthaltenen Informationen über die Abrechungsmodalitäten. Im Falle des vorliegenden Ausführungsbeispiels müsste der Kunde, falls er das Leistungsangebot des Leistungsanbieters mit Sitz in USA oder falls er das Leistungsangebot des Leistungsanbieters mit Sitz in Australien in Anspruch nehmen wollte, die Gesundheitsdienstleistung vollständig selbst bezahlen, da die Krankenkasse des Kunden die Kosten für die Erbringung dieser telemedizinischen Gesundheitsdienstleistung seitens eines Leistungsanbieters in USA und Australien nicht übernehmen würde. Da der Kunde eine vollständige Bezahlung der Kosten für die telemedizinische Gesundheitsdienstleistung gewünscht hat, wählt das Computerprogramm des Vertragsprüfungsmoduls 4 den Leistungsanbieter mit Sitz in Deutschland aus. Da in der Datenbank 5 alle für einen Vertragsschluss erforderlichen Daten des Leistungsanbieters mit Sitz in Deutschland vorhanden sind, also einerseits die Identifikationsdaten des Leistungsanbieters, welche zumindest den Namen, die Anschrift und die Kassenzulassung umfassen, und andererseits die Art und den Umfang der Leistung, also beispielsweise die Zurverfügungstellung eines Gerätes zur automatischen Messung der Blutgerinnungswerte sowie die Zusicherung der täglichen Überprüfung der Blutgerinnungswerte durch einen Facharzt, und zudem die Kundendaten vorhanden sind, kann das Computerprogramm des Vertragsprüfungsmoduls 4 automatisch einen Rohvertrag, also einen Vertragsentwurf, erzeugen, welcher auf die telemedizinische Gesundheitsdienstleistung mit dem Kunden und dem Leistungsanbieter als Vertragsparteien gerichtet ist.

Der Vertragsentwurf wird vorzugsweise automatisch, beispielsweise per E-Mail, über das Kommunikationsnetz 1 von dem Vertragsprüfungsmodul 4 an den Rechner 2 des Kunden und den Rechner 3 des Leistungsanbieters übermittelt, welche zum rechtkräftigen Abschluss des Vertrages miteinander Kontakt aufnehmen können. Bei dem Vertragsentwurf kann es sich gegebenenfalls auch bereits um den entgültigen Vertrag zwischen dem Kunden und dem Leistungsanbieter handeln, wenn beide Vertragsparteien mit dem Inhalt des Vertragsentwurfes einverstanden sind.

Es muss jedoch nicht notwendigerweise ein Rohvertrag von der Datenverarbeitungseinrichtung 4 erzeugt werden. Vielmehr können der Kunde, der Leistungsanbieter oder beide auch nur von dem Vermittler bzw. automatisiert durch die Datenverarbeitungseinrichtung 4, beispielsweise per E-Mail, informiert werden, so dass dies zu Vertragsverhandlungen miteinander Kontakt aufnehmen können.

Die Erfindung wurde vorstehend anhand einer telemedizinischen Gesundheitsdienstleistung für die Überwachung von Blutgerinnungswerten erläutert. Die Erfindung ist jedoch nicht auf diese oder gleichartige telemedizinische Gesundheitsdienstleistungen beschränkt. Vielmehr können mit dem erfindungsgemäßen Verfahren jegliche Formen telemedizinischer Gesundheitsdienstleistungen, seien diese diagnostischer oder therapeutischer Art, daraufhin überprüft werden, ob es zwischen einem Kunden und einem Leistungsanbieter einer derartigen telemedizinischen Gesundheitsdienstleistung zu einem Vertragsschluss für die Erbringung einer telemedizinischen Gesundheitsdienstleistung kommen kann.

Die Erfindung ermöglicht es demnach auch einer größeren medizinischen Einrichtung, welche über einen bestimmten Zeitraum eine bestimmte Anzahl von telemedizinischen Gesundheitsdienstleistungen pro Zeiteinheit und mit einer bestimmten Qualität erbracht haben möchte, einen geeigneten Vertragspartner für die Erbringung der telemedizinischen Gesundheitsdienstleistungen zu finden. Gemäß einem zweiten Ausführungsbeispiel sucht eine in USA ansässige auf die Behandlung von Diabetes spezialisierte Klinik eine Augenklinik, die innerhalb des nächsten halben Jahres pro Woche 200 Fundusbilder von Diabetespatienten auf diabetische Retinopathien untersuchen kann und dabei eine Sensitivität von 98% und eine Spezifität von 89% garantiert. Die Sensitivität sowie die Spezifität sind dabei Kriterien, anhand denen die Qualität der Untersuchungsergebnisse beurteilt werden kann. Die Sensitivität wird mit Hilfe bekannter Untersuchungsergebnisse ermittelt, wobei geprüft wird, wie viele der bekannten Untersuchungsergebnisse, welche eindeutig auf eine Krankheit oder Abnormalität hinweisen, erkannt werden. Bei der Spezifität wird anhand bekannter Untersuchungsergebnisse überprüft, wie viele der bekannten Untersuchungsergebnisse, welche eindeutig nicht auf eine Krankheit oder Abnormalität hinweisen, erkannt werden.

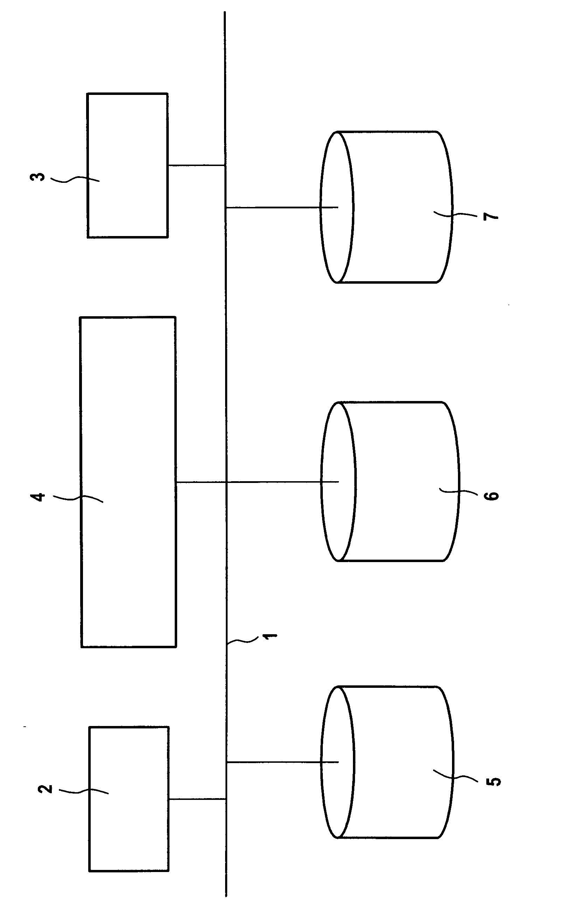

Wie zuvor beschrieben kann eine entsprechende Anfrage von der Klinik mit Hilfe eines Rechners, beispielsweise mit Hilfe des in der Figur gezeigten Rechners 2, an die Datenverarbeitungseinrichtung 4 gerichtet werden, die anhand der in der Anfrage enthaltenen Vorgaben einen geeigneten Leistungsanbieter zur Erbringung dieser telemedizinischen Gesundheitsdienstleitung durch Kontaktieren der Datenbank 5 sucht. Hat das Computerprogramm der Datenverarbeitungseinrichtung 4 einen oder mehrerer geeignete Leistungsanbieter innerhalb oder auch außerhalb der USA gefunden, werden auch in diesem Fall durch Abfragen der Datenbanken 6 und 7 die rechtlichen Gegebenheiten für die zu erbringende telemedizinische Gesundheitsdienstleistung sowie die abrechnungstechnischen Fragestellungen geklärt. Insbesondere wird im Falle des vorliegenden Ausführungsbeispiels überprüft, ob die Klinik bei Beauftragung eines der ermittelten Leistungsanbieters die Befundungsleistung selbst direkt mit den Krankenkasse der Patienten abrechnen kann oder ob die Klinik nur die Aufnahme der Fundusbilder mit der Krankenkasse der Patienten abrechnen kann und der Leistungsanbieter die Befundungsleistung selbst mit den Krankenkassen der Patienten abrechnen muss.

Sind diese Fragestellungen derart abgeklärt, dass ein Vertrag zwischen der Klinik und einem Leistungsanbieter zustande kommen kann, erzeugt das Computerprogramm des Vertragsprüfungsmoduls 4 vorzugsweise einen Rohvertrag, der den Parteien zugeht.

Sowohl im Falle des ersten als auch im Falle des zweiten Ausführungsbeispiels ist es möglich, dass das Computerprogramm des Vertragsprüfungsmoduls nach Zustandekommens eines Vertrags basierend auf dem Vertragsinhalt automatisch für die beiden an dem jeweiligen Vertrag beteiligten Parteien Arbeitsanweisungen und Leitlinien erstellt, wonach die Parteien für einen reibungslosen Erbringung der telemedizinischen Gesundheitsdienstleistung verfahren sollen.

Das Kommunikationsnetz, an das das Vertragsprüfungsmodul und die Datenbanken angeschlossen sind, muss im Übrigen nicht notwendigerweise das Internet sein. Vielmehr ist auch jedes andere öffentliche Kommunikationsnetz geeignet.

Des Weiteren kann es sich bei den Datenbanken 5 bis 7 auch um lokale Datenbanken handeln, auf die nur das Vertragsprüfungsmodul 4 Zugriff hat. Die Datenbanken können im Übrigen auch zu einer einzigen Datenbank zusammengefasst sein.

## Patentansprüche

1. Verfahren zu einer automatischen Überprüfung durch eine Datenverarbeitungseinrichtung (4), ob zwischen einem Leistungsanbieter einer telemedizinischen Gesundheitsdienstleistung und einem Kunden ein Vertrag über die telemedizinische Gesundheitsdienstleistung zustande kommen kann, aufweisend folgende Verfahrensschritte:
a) Annahme einer Anfrage eines Kunden zu einer telemedizinischen Gesundheitsdienstleistung durch eine an einem öffentlichen Kommunikationsnetz (1) angeschlossene Datenverarbeitungseinrichtung (4), welche Anfrage von einem Rechner (2) des Kunden über das öffentliche Kommunikationsnetz (1) an die Datenverarbeitungseinrichtung (4) übermittelt wird,
b) Kontaktierung einer ersten Datenbank (5) durch die Datenverarbeitungseinrichtung (4), welche Informationen über Leistungsangebote von Leistungsanbietern telemedizinischer Gesundheitsdienstleistungen enthält, und
c) Überprüfung durch die Datenverarbeitungseinrichtung (4), ob der Anfrage zu der telemedizinischen Gesundheitsdienstleistung ein im Wesentlichen entsprechendes Leistungsangebot eines Leistungsanbieters zugeordnet werden kann.

2. Verfahren nach Anspruch 1, bei dem die Datenverarbeitungseinrichtung (4) eine zweite Datenbank (6) kontaktiert, welche länderspezifische rechtliche Informationen zu telemedizinischen Gesundheitsdienstleistungen enthält.

3. Verfahren nach Anspruch 1 oder 2, bei dem die Datenverarbeitungseinrichtung (4) eine dritte Datenbank (7) kontaktiert, welche Informationen darüber enthält, welche telemedizinischen Gesundheitsdienstleistungen von Krankenkassen unter welchen Bedingungen zumindest teilweise bezahlt oder welche nicht bezahlt werden.

4. Verfahren nach einem der Ansprüche 1 bis 3, bei dem in dem Fall, dass zwischen einem Kunden und einem Leistungsanbieter ein Vertrag zustande kommen kann, zumindest ein Rohvertrag über die telemedizinische Gesundheitsdienstleistung durch die Datenverarbeitungseinrichtung (4) erzeugt wird.

5. Verfahren nach einem der Ansprüche 1 bis 4, bei dem die Anfrage eine medizinische Fragestellung und Identifikationsdaten des Kunden enthält.

6. Verfahren nach einem der Ansprüche 1 bis 5, bei dem das Leistungsangebot die Art und den Umfang der medizinischen Leistung sowie Identifikationsdaten des Anbieters enthält.

7. Verfahren nach einem der Ansprüche 1 bis 6, bei dem durch die Datenverarbeitungseinrichtung (4) durch Abfragen der ersten Datenbank (5) überprüft wird, ob ein Leistungsanbieter qualitative Vorgaben eines Kunden für die zu erbringende telemedizinische Gesundheitsdienstleitung einhalten kann.

8. Verfahren nach einem der Ansprüche 1 bis 7, bei dem durch die Datenverarbeitungseinrichtung (4) durch Abfragen der ersten Datenbank (5) überprüft wird, ob ein Leistungsanbieter quantitative Vorgaben eines Kunden für die zu erbringende telemedizinische Gesundheitsdienstleitung einhalten kann.

9. Verfahren nach einem der Ansprüche 1 bis 8, bei dem die Datenverarbeitungseinrichtung (4) nach Abschluss eines Vertrages basierend auf dem Vertragsinhalt Arbeitsanweisungen und/oder Leitlinien für den Kunden und den Leistungsanbieter erzeugt.

10. Verfahren nach einem der Ansprüche 1 bis 9, bei dem die Datenverarbeitungseinrichtung (4) ein Expertensystem ist.

11. Verfahren nach einem der Ansprüche 1 bis 10, bei dem die erste Datenbanken (5) an ein öffentliches Kommunikationsnetz (1) angeschlossen ist.

12. Verfahren nach einem der Ansprüche 2 bis 11, bei dem die zweite Datenbanken (6) an ein öffentliches Kommunikationsnetz (1) angeschlossen ist.

13. Verfahren nach einem der Ansprüche 3 bis 12, bei dem die dritte Datenbanken (7) an ein öffentliches Kommunikationsnetz (1) angeschlossen ist.
